(19) Europäisches Patentamt / European Patent Office / Office européen des brevets

(11) **EP 4 725 547 A2**

(12) **EUROPEAN PATENT APPLICATION**

(43) Date of publication:
**15.04.2026 Bulletin 2026/16**

(21) Application number: **26155932.2**

(22) Date of filing: **28.03.2022**

(51) International Patent Classification (IPC):
***A61P 17/00*** *(2006.01)*

(52) Cooperative Patent Classification (CPC):
**A61K 31/519; A61P 17/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2021 US 202163167833 P**

(62) Document number(s) of the earlier application(s) in
accordance with Art. 76 EPC:
**22714586.9 / 4 313 055**

(71) Applicant: **Pfizer Inc.
New York NY 10001 (US)**

(72) Inventor: **PEEVA, Elena
Cambridge, MA 02139 (US)**

(74) Representative: **Pfizer
European Patent Department
23-25 avenue du Docteur Lannelongue
75668 Paris Cedex 14 (FR)**

Remarks:
This application was filed on 03-02-2026 as a
divisional application to the application mentioned
under INID code 62.

(54) **METHODS FOR TREATMENT OF VITILIGO**

(57)    Method for treating vitiligo, including active and stable non-segmental vitiligo, using compounds and analogues which inhibit certain kinases including Janus Kinase (JAK), said method comprising the step of administering to the subject in need thereof 1-[(2S,5R)-2-methyl-5-(7H-pyrrolo[2,3-d]pyrimidin-4-ylamino)-1-piperidinyl]-2-propen-1-one, or a pharmaceutically acceptable salt thereof.

## Description

### FIELD OF THE INVENTION

The present invention provides methods for treating vitiligo using the compound 1-[(2S,5R)-2-methyl-5-(7H-pyrrolo[2,3-d]pyrimidin-4-ylamino)-1-piperidinyl]-2-propen-1-one, or a pharmaceutically acceptable salt thereof.

### BACKGROUND OF THE INVENTION

Protein kinases are families of enzymes that catalyze the phosphorylation of specific residues in proteins, broadly classified into tyrosine and serine/threonine kinases. Inappropriate kinase activity, arising from mutation, over-expression, or inappropriate regulation, dys-regulation or de-regulation, as well as over- or under-prod tion of growth factors or cytokines has been implicated in many diseases, including but not limited to cancer, cardiovascular diseases, allergies, asthma and other respiratory diseases, autoimmune diseases, inflammatory diseases, bone diseases, metabolic disorders, and neurological and neurodegenerative disorders such as Alzheimer's disease. Inappropriate kinase activity triggers a variety of biological cellular responses relating to cell growth, cell differentiation, cell function, survival, apoptosis, and cell mobility implicated in the aforementioned and related diseases.

Thus, protein kinases have emerged as an important class of enzymes as targets for therapeutic intervention. In particular, the JAK family of cellular protein tyrosine kinases (JAK1, JAK2, JAK3, and JAK3) play a central role in cytokine signaling (Kisseleva et al., Gene, 2002, 285, 1; Yamaoka et al. Genome Biology 2004, 5, 253)). Upon binding to their receptors, cytokines activate JAK which then phosphorylate the cytokine receptor, thereby creating docking sites for signaling molecules, notably, members of the signal transd er and activator of transcription (STAT) family that ultimately lead to gene expression. Numerous cytokines are known to activate the JAK family. These cytokines include, the interferon (IFN) family (IFN-alpha, IFN-beta, IFN-omega, Limitin, IFN-gamma, IL-10, IL-19, IL-20, IL-22), the gp130 family (IL-6, IL-11, OSM, LIF, CNTF, NNT-1/BSF-3, G-CSF, CT-1, Leptin, IL-12, IL-23), gamma C family (IL-2, IL-7, TSLP, IL-9, IL-15, IL-21, IL-4, IL-13), IL-3 family (IL-3, IL-5, GM-CSF), single chain family (EPO, GH, PRL, TPO), receptor tyrosine kinases (EGF, PDGF, CSF-1, HGF), and G-protein coupled receptors (AT1).

Vitiligo is an acquired hypopigmented disorder with or without autoimmune comorbidities including thyroid disease, alopecia areata, psoriasis, inflammatory bowel disease, type 1 diabetes mellitus and pernicious anemia. The worldwide prevalence of vitiligo ranges between 0.5% and 2.0%. Kruger, C. and K. U. Schallreuter (2012), Int J Dermatol 51(10): 1206 1212.

The trifecta of vitiligo pathogenesis can be oxidative stress causing melanocyte damage, genetics affecting melanocyte growth and differentiation, and autoimmunity involving autoreactive cytotoxic T cells, although, the complex pathophysiology of vitiligo is not fully understood. Stressed melanocytes may initiate the activated innate immunity via natural killer cells and nearby dendritic cells, followed by the activation of adaptive immunity. Picardo, M., et al., (2015), Nature Reviews Disease Primers 1: 15011. The presence of CD8+ T cells in close apposition to melanocytes suggests that the pathogenesis of vitiligo is T cell mediated although antibody-mediated pathogenesis is also proposed. Recent review proposes the hypothesis that CD8+ T cells produce IFN $\gamma$, which activate the release of CXCL9/10/11 from keratinocytes, which in turn activate the function of CD8+ T cells via the activation of CXCR3. Cytokines involved in vitiligo include IFNy, IL-2, IL-17, IL-15 and IL-4. Of note, IL-2 and IL-15 are suggested to activate CD49a+/CD8+ resident memory T cells in vitiligo skin and to induce perforin and granzyme B in those T cells, which induce melanocyte apoptosis. Cheuk, S., H., et al., (2017), Immunity 46(2): 287 300.

There are currently no approved treatments specifically to treat active non-segmental vitiligo. Current treatment options, which are limited and may be inefficacious, include topical corticosteroids, topical vitamin D3, topical calcineurin inhibitors, systemic corticosteroids (to treat rapidly progressive active lesions), phototherapy, surgical treatments (to treat small and stable lesions) and camouflage Ezzedine, K., V. et al. (2015), The Lancet 386(9988): 74 84; Taïeb, A. and M. Picardo (2009), New Eng J Med, 360(2): 160 169.

The compound 1-[(2S,5R)-2-methyl-5-(7H-pyrrolo[2,3-d]pyrimidin-4-ylamino)-1-piperidinyl]-2-propen-1-one, also generically known as ritlecitinib, is an orally bioavailable small molecule that inhibits, by irreversibly blocking the ATP binding site, JAK3 and the tyrosine kinase expressed in hepatocellular carcinoma (TEC) kinase family (bruton's tyrosine kinase [BTK], bone marrow expressed kinase [BMX], inducible T cell kinase [ITK], TEC, resting lymphocyte kinase [RLK/TXK]), with high selectivity over the other three JAK isoforms, JAK1, JAK2, and TYK2, as well as over the broader kinome.  1-[(2S,5R)-2-Methyl-5-(7H-pyrrolo[2,3-d]pyrimidin-4-ylamino)-1-piperidinyl]-2-propen-1-one  has  heretofore been investigated in patients with rheumatoid arthritis, alopecia areata, ulcerative colitis, and Crohn's disease.

Disclosed herein is the discovery that the compound 1-[(2S,5R)-2-methyl-5-(7H-pyrrolo[2,3-d]pyrimidin-4-ylamino)-1-piperidinyl]-2-propen-1-one, is useful for treating vitiligo, including active and stable non-segmental vitiligo. Accordingly, described herein are methods of reducing the severity of symptoms of vitiligo in a subject, with lower incidence of adverse effects.

## SUMMARY OF THE INVENTION

In a first aspect A1, the invention provides a method for treating vitiligo in a subject, comprising admistering to the subject in need thereof a therapeutically effective amount of 1-[(2S,5R)-2-methyl-5-(7H-pyrrolo[2,3-d]pyrimidin-4-ylamino)-1-piperidinyl]-2-propen-1-one or a pharmaceutically acceptable salt thereof.

## DETAILED DESCRIPTION OF THE INVENTION

Embodiments (E) of aspect A1 of the invention include the following, where for ease of subsequent reference, embodiment E1 is identical to aspect A1

E1. A method for treating vitiligo in a subject, comprising admistering to the subject in need thereof a therapeutically effective amount 1-[(2S,5R)-2-methyl-5-(7H-pyrrolo[2,3-d]pyrimidin-4-ylamino)-1-piperidinyl]-2-propen-1-one or a pharmaceutically acceptable salt thereof.

E2. The method according to embodiment E1, wherein the therapeutically effective amount is selected from the group consisting of 10, 30, 50, 100 or 200 mg QD.

E3. The method according to any one of embodiments E1 to E2, wherein the therapeutically effective amount is adminstered to the subject for an induction period of up to four weeks, or for a treatment period of up to 52 weeks.

E4. The method according to any one of embodiments E1 to E3, wherein the 1-[(2S,5R)-2-methyl-5-(7H-pyrrolo[2,3-d]pyrimidin-4-ylamino)-1-piperidinyl]-2-propen-1-one or a pharmaceutically acceptable salt thereof is administerd to the subject for a maintence period of up to 20 weeks following the induction period.

E5. The method according to any one of embodiments E1 to E4, wherein the vitiligo is non-segmented vitiligo.

E6. The method according to any one of embodiments E1 to E4 wherein the subject has a body surface area involvement fo 4- 50%.

E7. The method according to any one of embodiments E1 to E6, wherein the subject achieves a percent change from baseline for local read facial Vitiligo Area Scoring Index of at least about -15%.

E8. The method according to any one of embodiments E1 to E7, wherein the subject achieves a 75% reduction in F-VASI from baseline at week 24.

E9. The method according to any one of embodiments E1 to E8, wherein the subject achieves at least 4% reduction from baseline at week 8.

E10. The method according to any one of embodiments E1 to E9, wherein the subject achieves an at least 11% reduction from baseline at week 16.

E11. The method according to any one of embodiments E1 to E9, further comprising administering to the subject concurrent or subsequent treatment with narrow band ultraviolet B radiation add on therapy.

E12. The method according to any one of embodiments E11, wherin the narrow band ultraviolet B radiation add on therapy is administered for at least 24 weeks.

E13. The method according to any one of embodiments E12, whereby the subject achieves a percent change from baseline for local facial Vitiligo Area Scoring Index of up to between about -17% and about -30% at week 24 and between about -40% and about -80% at week 48.

E14. The method according to any one of embodiments E11 to E13, whereby the subject achieves a percent change from baseline for local facial Vitiligo Area Scoring Index of up to between about -17% and about -30% at week 24 and between about -40% and about -80% at week 48.

E15. The method according to any one of embodiments E11 to E14, whereby the subject achieves a percent change from baseline for total-Vitiligo Area Scoring Index of up to between about -5% and about -20% at week 24 and between about -30% and about -58% at week 48.

E16. The method according to any one of embodiments E1 to E15, wherein the therapeutically effective amount is 10 mg QD.

E17. The method according to any one of embodiments E1 to E15, wherein the therapeutically effective amount is 30 mg QD.

E18. The method according to any one of embodiments E1 to E15, wherein the therapeutically effective amount is 50 mg QD.

E19. The method according to any one of embodiments E1 to E15, wherein the therapeutically effective amount is 100 mg QD.

E20. The method according to any one of embodiments E1 to E15, wherein the therapeutically effective amount is 200 mg QD.

E21. The method according to any one of embodiments E1 to E15, wherein the therapeutically effective amount during the induction period is 50mg QD.

E22. The method according to any one of embodiments E1 to E15, wherein the therapeutically effective amount during the induction period is 100 mg QD.

E23. The method according to any one of embodiments E1 to E15, wherein the therapeutically effective amount during the induction period is 200 mg QD.

E24 The method according to any one of embodiments E1 to E23, wherein the therapeutically effective amount during the maintenance period is 10 mg QD.

E25. The method according to any one of embodiments E1 to E23, wherein the therapeutically effective amount during the maintenance period is 30 mg QD.

E26. The method according to any one of embodiments E1 to E23, wherein the therapeutically effective amount for the maintenance period is 30 mg QD.

E27. The method according to any one of embodiments E1 to E23, wherein the therapeutically effective amount during the maintenance period is 50 mg QD.

E28. The method according to any one of embodiments E1 to E15, wherein the therapeutically effective amount during the induction period is 100 mg and, the therapeutically effective amount during the induction period during the maintenance period is 50 mg QD.

E29. The method according to any one of embodiments E1 to E15, wherein the therapeutically effective amount during the induction period is 200 mg and the therapeutically effective amount during the maintenance period is 50 mg QD.

E30. The method according to any one of embodiments E1 to E29, wherein the 1-[(2S,5R)-2-methyl-5-(7H-pyrrolo[2,3-d]pyrimidin-4-ylamino)-1-piperidinyl]-2-propen-1-one is a free base.

E31. Use of the compound 1-[(2S,5R)-2-methyl-5-(7H-pyrrolo[2,3-d]pyrimidin-4-ylamino)-1-piperidinyl]-2-propen-1-one, or a pharmaceutically acceptable salt thereof, for the preparation of a medicament for the treatment of vitiligo according to any one of embodiments E1 to E30.

E32. The compound 1-[(2S,5R)-2-methyl-5-(7H-pyrrolo[2,3-d]pyrimidin-4-ylamino)-1-piperidinyl]-2-propen-1-one, or a pharmaceutically acceptable salt thereof, for use in a method of treating vitiligo according to any one of embodiments E1-E30.

In therapeutic use for treating vitiligo in a subject, ritlecitinib and compounds of the present invention or its pharmaceutical compositions can be administered orally, parenterally, topically, rectally, transmucosally, or intestinally. Parenteral administrations include indirect injections to generate a systemic effect or direct injections to the afflicted area. Topical administrations include the treatment of skin or organs readily accessible by local application, for example, peri-orbitally to the eyes or to the ears. It also includes transdermal delivery to generate a systemic effect. The rectal administration includes the form of suppositories. The preferred routes of administration are oral and topical.

Pharmaceutical compositions used in the present invention may be manufactured by methods well known in the art, e.g., by means of conventional mixing, dissolving, granulation, dragee-making, levigating, emulsifying, encapsulating, entrapping, lyophilizing processes or spray drying.

Pharmaceutical compositions for use in accordance with the present invention may be formulated in conventional manner using one or more pharmaceutically acceptable carriers comprising excipients and auxiliaries, which facilitate processing of the active compound into preparations, which can be used pharmaceutically. Proper formulation is dependent upon the route of administration chosen. Pharmaceutically acceptable excipients and carriers are generally known to those skilled in the art and are thus included in the instant invention. S h excipients and carriers are described, for example, in Remington's Pharmaceutical Sciences, Mack Pub. Co., New Jersey (1991). The formulations used in the invention can be designed to be short-acting, fast-releasing, long-acting, and sustained-releasing. Thus, the pharmaceutical formulations can also be formulated for controlled release or for slow release.

Also, it is to be understood that the initial dosage administered may be increased beyond the above upper level in order to rapidly achieve the desired plasma concentration. On the other hand, the initial dosage may be smaller than the optimum and the daily dosage may be progressively increased during the course of treatment depending on the particular situation. If desired, the daily dose may also be divided into multiple doses for administration, e.g., two to four times per day.

The compounds used in practice of the invention may be prepared by any method known in the art. In particular, the compounds can be prepared by the procedures described by reference to the prior art references in which they are disclosed.

The prepration for ritlecitinib,1-[(2S,5R)-2-methyl-5-(7H-pyrrolo[2,3-d]pyrimidin-4-ylamino)-1-piperidinyl]-2-propen-1-one, are disclosed in US Patent No. 10,144,738, the contents of which are incorporated herein in their entirety. Formulations for ritlecitinib,1-[(2S,5R)-2-methyl-5-(7H-pyrrolo[2,3-d]pyrimidin-4-ylamino)-1-piperidinyl]-2-propen-1-one, are disclosed in US Provisional Patent Application Serial No. 62/949995, the contents of which are incorporated herein in their entirety.

As used herein, a "patient", an "individual" or a "subject", used interchangeably herein, is a mammal, more preferably, a human. Mammals also include, but are not limited to, farm animals (e.g., cows, pigs, horses, chickens, etc.), sport animals, pets, primates, horses, dogs, cats, mice and rats.

As used herein, an "induction period" is an initial period of treatment, typically, of up to about 4 to 8 weeks in which one dose is administered, usually, at a higher relative dose of a therapeutic drug and is prior to a "maintenance period" in which another typically higher dose is administered.

The term "adverse effect" (AE) is any untoward medical occurrence in a subject or clinical study participant, temporally associated with the administration of the JAK inhibitor.

The term "QD" or "Q.D." means one administered dose per day.

The term "treating" or "treatment" means an alleviation of symptoms associated with a disease, disorder or condition, or halt of further progression or worsening of those symptoms. Depending on the disease and condition of the subject, the term "treatment" as used herein may include one or more of curative, palliative and prophylactic treatment. Treatment can also include administering a pharmaceutical formulation of the present invention in combination with other therapies.

The term "therapeutically effective" indicates the capability of an agent to prevent, or improve the severity of the disorder, while avoiding adverse side effects typically associated with alternative therapies. The phrase "therapeutically effective" is to be understood to be equivalent to the phrase "effective for the treatment, prevention, or amelioration", and both are intended to qualify the amount of each agent for use in the combination therapy which will achieve the goal of improvement in the severity of disease, or pain or other symptom thereof, and the frequency of incidence over treatment of each agent by itself, while avoiding adverse side effects typically associated with alternative therapies.

"Pharmaceutically acceptable" means suitable for use in a "subject."

## EXAMPLES

The following non-limiting examples are presented merely to illustrate the present invention. The skilled person will understand that there are numerous equivalents and variations not exemplified but which still form part of the present teachings.

### Example 1

### Methods of Treatment of Vitiligo

Subjects take the medication set forth herein orally once daily, and typically swallow the tablets whole with approximately one cup of ambient temper ature water, without any manipulation or chewing. Dosing regimes include induction dosing of 100 or 200mg and maintenance dosing of 50 mg, or a 24-week Treatment Period in which participants were treated with ritlecitinib 50 mg, 30 mg, or 10 mg. The induction period is an initial period of treatment of 4 weeks in the initial clinical trial. The maintence period is 20 weeks Tablets may be taken with or without food. Subject response is evaluated at regular intervals after administration. The Vitiligo Area Scoring Index (VASI) is a validated quantitative scale, initially developed to measure the response of vitiligo to nbUVB treatment, but has since been used to evaluate various therapies for vitiligo. Clinical evaluations of vitiligo in this study include VASI, central read and site assessment of facial-VASI, static Investigator Global Assessment (sIGA), Body Surface Area (BSA) involvement, Vitiligo Extent Score (VES), and dermoscopy. Self-Assessment VES (SA-VES), Vitiligo Specific Quality of Life (VitiQoL), Dermatology Life Quality Index (DLQL), Hospital Anxiety and Depression Scale (HADS), Patient Global Impression of Change Vitiligo (PGIC-V), EQ-5D-5L, Healthcare Resource Utilization (HCRU) and Vitiligo Noticeability Scale (VNS).

### Method of Evaluation

### (a) nbUVB phototherapy

The nbUVB phototherapy administration follows the Vitiligo Working Group phototherapy recommendations except that the nbUVB phototherapy is conducted twice a week during the study instead of three times a week. Mohammad, T. F., et al. (2017), J Am Acad Dermatol 76(5): 879-888.

### (b) Facial Vitiligo Area Scoring Index (Facial-VASI)-Central Read

The central read facial-VASI are assessed based on the facial photographs taken at the site by a central reader. Clinical Trial Imaging Endpoint Process Standards - Guidance for Industry (April 2018).

The central read facial-VASI is calculated using a formula that includes contribution of affected facial surface areas showing all six (6) different depigmentation rates (0.1, 0.25, 0.5, 0.75, 0.9 and 1) with a modified method described by Hamzavi et al. Hamzavi, I., $eA = \pi r^2$

The calculation of % affected total body surface area (BSA) is shown below:

Affected Facial Surface Area X 4 = % Affected Total Body Surface Area.

Face is defined as the area from the hairline on top of the forehead to the jawline at the bottom of the cheeks. Facial-VASI (central read) will range from 0.000 to 4.000 by defining the affected Facial Surface Area (expressed as the value between 0.0 to 1.0) being 4% of total Body Surface Area.

For example: 25% affected Facial Surface Area (0.25) represents 1% total body surface area (0.25 X 4). 15% affected Facial Surface Area (0.15) represents 0.6% total body surface area (0.15 X 4).

The extent of depigmentation is expressed by the following percentages: 0, 10%, 25%, 50%, 75%, 90%, or 100%. At 100% depigmentation, no pigment is present; at 90%, specks of pigment are present; at 75%, the depigmented exceeded the pigmented area; at 50%, the depigmented and pigmented areas are equal; at 25%, the pigmented area exceeded the depigmented area; and at 10%, only specks of depigmentation are present.

Scalp, neck, eyebrows, eyelashes, and vermilion are excluded from this calculation, although total VASI assessment includes all of these regions.

Ritlecitinib met the efficacy criterion for the primary endpoint, as shown by initial clinical studies. Data are set forth in the Table below:

**Primary Efficacy Endpoint** - **% CFB in Central F-VASI at Week 24**

| Treatment group | Placebo-adjusted LS mean CFB (SE) | 90% CI | Adjusted p-value | Unadjusted p-value |
|---|---|---|---|---|
| 200/50 mg | -23.62 (5.79) | (-33.18, -14.05) | <0.0001 | - |
| 100/50 mg | -22.99 (5.74) | (-32.46. -13.51) | <0.0001 | - |
| 50 mg | -22.05 (6.00) | (-31.96, -12.14) | 0.0001 | - |
| 30 mg | -16.93 (6.87) | (-28.26, -5.59) | - | 0.0072 |
| 10 mg | -4.13 (6.16) | (-14.30, 6.04) | - | 0.2516 |

(c) **Total-Vitiligo Area Scoring Index** (VASI)

The total body VASI scores are assessed by the Investigator. The total body VASI are calculated using a formula that includes contribution from all body regions (possible range, 0-100) with a modified method described by Hamzavi et al.:

$$Total - VASI = \sum_{Six\ Different\ Body\ Sites} [Hand\ Units]\ X\ [Depigmentation]$$

Percent of total body surface area (BSA) is determined by hand units. One hand unit, which encompasses the palm plus the volar surface of all the digits, is approximately 1% of the total body surface area and is used as a guide to estimate the baseline percentage of vitiligo involvement of each body region.

The body are divided into 6 separate and mutually exclusive regions: face/neck, hands, upper extremities (excluding hands), trunk, lower extremities (excluding the feet), and feet. The axillary regions are included with the upper extremities, while the buttocks and inguinal regions are included with the lower extremities. Genital area is included in trunk. Face and neck lesions are measured in this study.

The extent of depigmentation is expressed by the following percentages: 0, 10%, 25%, 50%, 75%, 90%, or 100%. At 100% depigmentation, no pigment is present; at 90%, specks of pigment are present; at 75%, the depigmented exceeded the pigmented area; at 50%, the depigmented and pigmented areas are equal; at 25%, the pigmented area exceeded the depigmented area; and at 10%, only specks of depigmentation are present.

(d) **Facial Vitiligo Area Scoring Index (Facial-VASI)-Site Assessment**

The site assessed facial-VASI are assessed by the Investigator. The site assessment of the facial-VASI is calculated using a formula that is similar to facial-VASI central read. It includes contribution from face (possible range, 0.00-4.00). Scalp, neck, eyebrows, eyelashes, and vermilion are excluded from this calculation, although total VASI assessment includes all of these regions.

$$Facial\ VASI\ =[Digit\ Units]\ X\ [Depigmentation]\ X\ 0.1$$

The volar surface of one digit (the subject's thumb) is approximately 0.1% of the total body surface area and was used as a guide to estimate the baseline percentage of vitiligo involvement of face.

The extent of depigmentation is expressed by the following percentages: 0, 10%, 25%, 50%, 75%, 90%, or 100%. At 100% depigmentation, no pigment is present; at 90%, specks of pigment are present; at 75%, the depigmented exceeded the pigmented area; at 50%, the depigmented and pigmented areas are equal; at 25%, the pigmented area exceeded the depigmented area; and at 10%, only specks of depigmentation are present.

**(e) Static Investigator Global Assessment (sIGA)**

**Static Investigator Global Assessment (sIGA) Score**

| Score | Short descriptor | Detailed descriptor |
|---|---|---|
| 0 | Clear | • No signs of loss of pigmentation with natural light or with Woods lamp examination. |
| 1 | Almost clear | • Faint, barely detectable loss of pigmentation mainly located on dorsal hands, feet, bony prominences, and/or limited areas.<br>• Approximately 90% pigmentation within lesions.<br>• No or rare signs of Koebner phenomenon, confetti-like or trichrome lesions may be present. |
| 2 | Mild vitiligo | • Mild loss of pigmentation mainly located on dorsal hands, feet, bony prominences, and/or limited areas.<br>• Approximately 75% pigmentation within lesions.<br>• Few signs of Koebner phenomenon, confetti-like or trichrome lesions may be present. |
| 3 | Moderate vitiligo | • Moderate loss of pigmentation affecting several areas of the body with large patches.<br>• Approximately 50% pigmentation within lesions.<br>• Moderate number of signs of Koebner phenomenon, confetti-like or trichrome lesions may be present. |
| 4 | Severe vitiligo | • Extensive loss of pigmentation affecting most areas of the body.<br>• Approximately 25% or less pigmentation within lesions.<br>• Many signs of Koebner phenomenon, confetti-like or trichrome lesions affecting several areas of the body may be present. |

**(f) Body Surface Area (BSA)**

The number of hand units of skin afflicted with vitiligo in a body region can be used to determine the extent (%) to which a body region is involved with vitiligo. When measuring, the hand unit refers to the size of each individual subject's palm plus the volar surface of all the digits in a closed position. Rule of 9 is used to estimate BSA (Head/neck 9%; Upper extremities excluding hands 14%; Hands 4%; trunk including genital area 33%; Lower extremities excluding feet 36%; Feet 4%).

**(g) Vitiligo Extent Score (VES)**

Vitiligo Extent Score is a measure to express the overall vitiligo involvement of the body (extent). van Geel, N., et al., J Am Acad Dermatol 76(3): 464-471.

Clinical illustrations for 19 separate body areas that reflect different degrees of involvement (1, 5, 10, 25, 50, and 75% depigmentation) are chosen to represent the subject's skin lesions to get the total extent of the disease. VES is a sum of all surface measurement that is similar to VASI.

**(h) Dermoscopy**

At least two different anatomical regions based on VES are selected on Screening. At least two fields (possibly peripheral and central) from the largest lesion in the selected anatomical region are examined to determine if white hair is present in less than 30% of hair in the depigmented lesion. Number of fields with less than 30% of white hair are recorded. The same chosen lesions are assessed. The same dermoscopy is used throughout the study.

**(i) Target Lesion(s) Assessment**

One isolated and completed stable lesion (if applicable) are identified as the stable target lesion on Day 1. Photographs of the lesion are obtained (according to the separately provided Photography Instructions) at various time points. The extent of depigmentation of the target lesion is expressed by the following percentages: 0, 10%, 25%, 50%, 75%, 90%, or 100%. At 100% depigmentation, no pigment is present; at 90%, specks of pigment are present; at 75%, the depigmented exceeds the pigmented area; at 50%, the depigmented and pigmented areas are equal; at 25%, the pigmented area exceeded the depigmented area; and at 10%, only specks of depigmentation are present. The same target lesion are assessed.

In case of mixed vitiligo, one isolated segmental vitiligo lesion (if applicable) are identified as the segmental target lesion on Day 1. Photographs of the lesion are obtained (according to the separately provided Photography Instructions) at various time points. The extent of depigmentation of the target lesion is expressed by the following percentages: 0, 10%, 25%, 50%, 75%, 90%, or 100%. At 100% depigmentation, no pigment is present; at 90%, specks of pigment are present; at 75%, the depigmented exceeds the pigmented area; at 50%, the depigmented and pigmented areas are equal; at 25%, the pigmented area exceeded the depigmented area; and at 10%, only specks of depigmentation are present. The same target lesion are assessed.

Target lesion assessment for active vitiligo lesion is not required. Photographs of active lesion, stable lesion, and segmental vitiligo lesion (if applicable) are obtained.

**(j) Patient Report Outcome (PRO) Measures**

Every effort should be made to have the subject complete all patient reported outcome (PRO) questionnaires before any other evaluations. All PROs should be completed in the order specified in as following: SA-VES, VitiQoL, DLQI, PHQ-8, PGIC-V, and VNS, at those visits where they are to be administered except for Screening Visit and Baseline Visit. All of the PROs are administered as electronic version on a tablet with the exception of the VNS which are administered as a paper version.

**(k) Self-Assessment Vitiligo Extent Score (SA-VES)**

The Self-Assessment Vitiligo Extent Score (SA-VES) is a validated patient report outcome measurement instrument to provide information about disease extent.

**(l) Vitiligo-Specific Quality of Life (VitiQoL) and Patient Global Impression of Severity of Vitiligo (PGIS-V)**

The Vitiligo-Specific Quality of Life Instrument (VitiQoL) is a reliable and validated vitiligo disease-specific HRQoL instrument which measures concepts relevant to vitiligo subjects. The VitiQoL is a 15-item PRO measure which measures concepts of symptoms, daily activities, leisure activities, work, personal relationships and treatment. Responses range from "not at all" (scored 0) to "most of the time" (scored 6) and gives a minimum and maximum score from 0-90, with higher scores representing greater burden. A minimally important difference has not yet been established for the questionnaire.

The Patient Global Impression of Severity of Vitiligo (PGIS-V) is 1-item within the VitiQoL questionnaire which asks the subject to determine how severe they feel their skin condition is at that point in time on a 7-point Likert response from "no skin involvement" to "most severe case."

**(m) Dermatology Life Quality Index (DLQI)**

The DLQI is a general dermatology questionnaire that consists of 10 items that assess subject health-related quality of life (daily activities, personal relationships, symptoms and feelings, leisure, work and school, and treatment). The DLQI is a psychometrically valid and reliable instrument that has been translated into several languages, and the DLQI total scores have been shown to be responsive to change. The minimal clinically important difference for the DLQI has been estimated as a 2 to 5 point change from baseline.

**(n) Vitiligo Noticeability Scale (VNS)**

The Vitiligo Noticeability Scale is a 1-item patient completed scale that assesses noticeability of vitiligo patches "now" compared with before treatment - as a way of evaluating treatment effect from the patient perspective. Batchelor, et al., Br. J. Derm. (2016), 174: 386-394. The scale includes 5 point Likert options ranging from 1= more noticeable to 5= no longer noticeable. Patients complete this scale and are required to review their Day 1 facial images (before treatment) and look at their face in the mirror the day of which the scale is completed to complete their response on this scale.

**Claims**

1.  A method for treating vitiligo in a subject, comprising administering to the subject in need thereof a therapeutically effective amount 1-[(2S,5R)-2-methyl-5-(7H-pyrrolo[2,3-d]pyrimidin-4-ylamino)-1-piperidinyl]-2-propen-1-one or a pharmaceutically acceptable salt thereof.

2.  The method of claim 1, wherein the therapeutically effective amount is selected from the group consisting of 10, 30, 50, 100 or 200 mg QD.

3.  The method of any one of claims 1 to 2, wherein the 1-[(2S,5R)-2-methyl-5-(7H-pyrrolo[2,3-d]pyrimidin-4-ylamino)-1-piperidinyl]-2-propen-1-one or a pharmaceutically acceptable salt thereof is administered to the subject for a maintenance period of up to 20 weeks following the induction period.

4.  The method of any one of claims 1 to 3, wherein the subject has a body surface area involvement fo 4- 50%.

5.  The method of any one of claims 1 to 3, wherein the subject achieves a percent change from baseline for local read facial Vitiligo Area Scoring Index of at least about -15%.

6.  The method of any one of claims 1 to 3, wherein the subject achieves a 75% reduction in F-VASI from baseline at week 24.

7.  The method of any one of claims 1 to 3, further comprising administering to the subject concurrent or subsequent treatment with narrow band ultraviolet B radiation add on therapy.

8.  The method of claim 7, wherin the narrow band ultraviolet B radiation add on therapy is administered for at least 24 weeks.

9.  The method of claim 7, whereby the subject achieves a percent change from baseline for local facial Vitiligo Area Scoring Index of up to between about -17% and about -30% at week 24 and between about -40% and about -80% at week 48.

10. The method of any one of claims 1 to 9, wherein the therapeutically effective amount is 10 mg QD.

11. The method according to any one of claims 1 to 9, wherein the therapeutically effective amount is 30 mg QD.

12. The method of any one of claims 1 to 9, wherein the therapeutically effective amount is 50 mg QD.

13. The method of any one of claims 1 to 9, wherein the therapeutically effective amount is 100 mg QD.

14. The method of any one of claims 1 to 9, wherein the therapeutically effective amount during the induction period is 50mg QD.

15. The method of any one of claims 1 to 9, wherein the therapeutically effective amount during the induction period is 100 mg QD.

16. Use of the compound 1-[(2S,5R)-2-methyl-5-(7H-pyrrolo[2,3-d]pyrimidin-4-ylamino)-1-piperidinyl]-2-propen-1-one, or a pharmaceutically acceptable salt thereof, for the preparation of a medicament for the treatment of vitiligo according to any one of claims 1 to 15.

17. The compound 1-[(2S,5R)-2-methyl-5-(7H-pyrrolo[2,3-d]pyrimidin-4-ylamino)-1-piperidinyl]-2-propen-1-one, or a pharmaceutically acceptable salt thereof, for use in a method of treating vitiligo according to any one of claims 1 to 15.

## REFERENCES CITED IN THE DESCRIPTION

**Patent documents cited in the description**

- US 10144738 B
- US 62949995

**Non-patent literature cited in the description**

- **KISSELEVA et al.** *Gene*, 2002, vol. 285, 1
- **YAMAOKA et al.** *Genome Biology*, 2004, vol. 5, 253
- **KRUGER, C.** ; **K. U. SCHALLREUTER**. *Int J Dermatol*, 2012, vol. 51 (10), 1206-1212
- **PICARDO, M. et al.** *Nature Reviews Disease Primers*, 2015, vol. 1, 15011
- **CHEUK, S., H. et al.** *Immunity*, 2017, vol. 46 (2), 287-300
- **EZZEDINE, K., V. et al.** *The Lancet*, 2015, vol. 386 (9988), 74-84
- **TAÏEB, A.** ; **M. PICARDO**. *New Eng J Med*, 2009, vol. 360 (2), 160-169
- Remington's Pharmaceutical Sciences. Mack Pub. Co, 1991
- **MOHAMMAD, T. F. et al.** *J Am Acad Dermatol*, 2017, vol. 76 (5), 879-888
- *Clinical Trial Imaging Endpoint Process Standards - Guidance for Industry*, April 2018
- **VAN GEEL, N. et al.** *J Am Acad Dermatol*, vol. 76 (3), 464-471
- **BATCHELOR et al.** *Br. J. Derm.*, 2016, vol. 174, 386-394